# EUROPEAN PATENT APPLICATION

(11) **EP 4 603 478 A1**
(43) Date of publication of application: **20.08.2025**
(21) Application number: 23892048.2
(22) Date of filing: 16.11.2023
(51) Int. Cl.: C07C 263/10, C07C 265/04, C08G 18/38, C08G 18/76, C08L 75/04, G02B 1/04

(54) **XYLENE DIISOCYANATE COMPOSITION AND PREPARATION METHOD THEREFOR**

(30) Priority: 16.11.2022 KR 20220153715
(71) Applicant: Hanwha Solutions Corporation, Jung-gu Seoul 04541 (KR)
(72) Inventor: SIM, Yujin, Daejeon 34128 (KR); PARK, Juyoung, Daejeon 34128 (KR); PARK, Jongseong, Daejeon 34128 (KR); WOO, Eun Ji, Daejeon 34128 (KR)
(74) Representative: Berggren Oy
(86) International application number: PCT/KR2023/018490
(87) International publication number: WO 2024/106985

(57) **Abstract**

Provided is a technology regarding to a xylylene diisocyanate composition in which the content of low-boiling-point compounds is controlled, and a preparation method thereof. Provided is a technology regarding to a xylylene diisocyanate composition capable of preparing a high-purity xylylene diisocyanate compound in a high yield by controlling purity of an amine compound and controlling the content range of low-boiling-point compounds in a reactant upon preparing xylylene diisocyanate using phosgene, and a preparation method thereof.

## Description

### [Technical Field]

### Cross-reference to Related Application

The present application is based on, and claims priority from, Korean Patent Application Nos. 10-2022-0153715 and 10-2023-0159228, filed on November 16, 2022 and November 16, 2023, respectively, the disclosures of which are hereby incorporated by reference herein in their entirety.

The present invention relates to a xylene diisocyanate composition in which the content of low-boiling-point compounds is controlled, and a preparation method thereof.

### [Background Art]

Although xylylene diisocyanate(hereinafter, referred to as XDI) includes an aromatic ring, it is classified into aliphatic isocyanate, and is a compound very useful as raw materials such as polyurethane-based materials, polyurea-based materials, or polyisocyanurate-based materials in the fields of chemical industry, resin industry, and paint industry.

Commonly, aliphatic isocyanate is prepared by a phosgenation method of reacting a raw material amine with phosgene. For example, XDI is prepared by reacting xylylene diamine (hereinafter, referred to as XDA) with phosgene. However, since XDI have high reactivity of the amino group, similar to the characteristics of aliphatic isocyanate, many side reactions are generated during the phosgenation reaction, and impurities generated through the side reactions have an influence on a process of producing a polyurethane resin to generate a problem of quality deterioration of the resin.

Therefore, many methods of preparing high-purity XDI by reducing the content of the generated impurities have been studied and proposed.

Specifically, Korean Patent Publication No. 1994-0001948 discloses a method of preparing high-purity XDI by using an ester-based compound such as amyl acetate or hexyl acetate as a reaction solvent during preparation of xylene diisocyanate by a reaction of xylene diamine or a hydrochloride thereof with phosgene. However, this method is problematic in that the solvent is expensive and purity and yield are still low.

Further, Korean Patent No. 0953019 discloses a method of preparing isocyanate by a phosgenation reaction of amine hydrochloride which is prepared by a salt-forming process of reacting a linear or cyclic aliphatic amine with hydrogen chloride, wherein a pressure is applied during the salt-forming process in order to solve the transfer problem of amine hydrochloride.

As a non-phosgenation method of using no phosgene, Korean Patent No. 1318828 discloses a method of preparing xylene diisocyanate through a non-phosgenation method, wherein a diamine compound is reacted with alkyl chloroformate or dialkyl carbonate to prepare biscarbamate, which is then subjected to thermal decomposition, thereby degrading and removing alcohol having a relatively low boiling point. However, as compared with the phosgenation method, this method is disadvantageous in terms of cost, and its application to industrial mass-production is difficult.

### [Disclosure]

### [Technical Problem]

There is provided a technology regarding to a xylene diisocyanate composition in which the content of low-boiling-point compounds is controlled, and a preparation method thereof.

There is provided a method of preparing xylene diisocyanate, the method capable of preparing a high-purity xylene diisocyanate compound in a high yield by controlling purity of an amine compound and controlling the content range of particular low-boiling-point compounds in a reactant upon preparing xylene diisocyanate using phosgene.

There is also provided a preparation method capable of economically preparing a high-purity xylene diisocyanate compound, in which a salt-forming reaction is performed under specific conditions to utilize the reaction heat generated in the corresponding reaction, thereby increasing the process efficiency of a phosgene reaction.

### [Technical Solution]

According to one embodiment of the present invention, there is provided a xylene diisocyanate composition including
a xylene diisocyanate compound; and
low-boiling-point compounds including isocyanomethylbenzaldehyde and isocyanomethylbenznitrile,
wherein the low-boiling-point compounds are included in an amount of 1% or less with respect to the total amount of the composition.

According to one embodiment of the present invention, there is provided a method of preparing a xylene diisocyanate composition, the method including:
a first step of reacting an amine compound with hydrogen chloride in a solvent at 20°C to 90°C under normal pressure to obtain an amine salt compound;
a second step of reacting the amine salt compound with phosgene to obtain a reaction mixture including a xylene isocyanate compound; and
a third step of removing the solvent and unreacted phosgene from the reaction mixture to prepare the xylene diisocyanate composition,
wherein the solvent is one or more selected from the group consisting of chlorobenzene, 1,2-dichlorobenzene, 1,4-dichlorobenzene, 1,2,3-trichlorobenzene, and 1,2,4-trichlorobenzene,
a purity of the amine compound is 99.0% or more, and
the xylene diisocyanate composition includes low-boiling-point compounds of 1% or less with respect to the total amount of the composition, and the low-boiling-point compounds include isocyanomethylbenzaldehyde and isocyanomethylbenznitrile.

According to another embodiment of the present invention, there is provided a method of preparing a polyisocyanate composition, the method including:
a first step of reacting an amine compound with hydrogen chloride in a solvent at 20°C to 90°C under normal pressure to obtain an amine salt compound;
a second step of reacting the amine salt compound with phosgene to obtain a reaction mixture including a xylene diisocyanate compound;
a third step of removing the solvent and unreacted phosgene from the reaction mixture to prepare the xylene diisocyanate composition; and
a fourth step of polymerizing the xylene diisocyanate composition and polyhydric alcohol to synthesize the polyisocyanate compound,
wherein the solvent is one or more selected from the group consisting of chlorobenzene, 1,2-dichlorobenzene, 1,4-dichlorobenzene, 1,2,3-trichlorobenzene, and 1,2,4-trichlorobenzene,
a purity of the amine compound is 99.0% or more, and
the xylene diisocyanate composition includes low-boiling-point compounds of 1% or less with respect to the total amount of the composition, and the low-boiling-point compounds include isocyanomethylbenzaldehyde and isocyanomethylbenznitrile.

According to still another embodiment of the present invention, there is provided a polymerizable composition including
the above-described xylene diisocyanate composition; and
any one or more of i) a polyfunctional thiol-based compound and ii) a polyfunctional episulfide compound.

According to still another embodiment of the present invention, there is provided an optical article including a polythiourethane polymer which is prepared from the polymerizable composition.

### [Effect of the Invention]

A xylene diisocyanate composition according to the present invention may prepare a high-purity polyisocyanate compound in a high yield by controlling the content range of particular low-boiling-point compounds without generating additional impurities when synthesizing polyisocyanate.

Further, a method of preparing the xylene diisocyanate composition according to the present invention may prepare a high-purity xylene diisocyanate compound in a high yield through a simple preparation process of controlling purity of the reaction composition, and at the same time, controlling the content range of particular low-boiling-point compounds among by-products. In addition, the method of preparing the xylene diisocyanate composition according to the present invention may economically prepare a high-purity xylene diisocyanate compound by controlling salt-forming reaction conditions of an amine compound within an appropriate range to increase process efficiency in a subsequent phosgenation reaction.

In addition, a method of preparing a polyisocyanate composition according to the present invention may prepare a high-purity polyisocyanate compound in a high yield by controlling purity of the reaction composition, and at the same time, controlling the content range of particular low-boiling-point compounds among by-products.

### [Best Mode for Carrying Out the Invention]

The terms used in this description are just for explaining exemplary embodiments and it is not intended to restrict the present invention. The singular expression may include the plural expression unless it is differently expressed contextually. It must be understood that the term "comprise", "equipped", or "have" in the present description is only used for designating the existence of characteristics taken effect, numbers, steps, components, or combinations thereof, and do not exclude the existence or the possibility of addition of one or more different characteristics, numbers, steps, components or combinations thereof beforehand.

While the present invention is susceptible to various modifications and alternative forms, specific embodiments will be illustrated and described in detail as follows. It should be understood, however, that the description is not intended to limit the present invention to the particular forms disclosed, but on the contrary, the intention is to cover all modifications, equivalents, and alternatives falling within the spirit and scope of the invention.

Commonly, preparation of an isocyanate compound using a phosgenation reaction is carried out by a reaction of an amine compound and phosgene, and at this time, various impurities are generated as by-products. The present inventors have determined the influence of the application of polyisocyanate, which is polymerized using isocyanate including such various impurities, to actual products, and have confirmed that high-purity isocyanate may be prepared in a high yield by controlling the purity of a solvent and an amine compound which are reactants, and at the same time, by controlling the content of low-boiling-point compounds in the reaction mixture to a specific range (about 1% or less), thereby completing the present invention.

**In** particular, low-boiling-point compounds among the reaction by-products generate additional impurities during the synthesis of polyisocyanate, and interfere with activity of a catalyst to affect the reaction rate, thereby affecting quality of a product. However, by controlling the content to an appropriate range as described above, it is possible to prepare a product of excellent quality without such problems, and such a high-purity polyisocyanate compound may remarkably reduce the defect rate and may improve reproducibility when applied to optical articles.

In addition, the amine compound used in the phosgenation reaction is prepared in the form of a chloride through a salt-forming reaction. In the present invention, the salt-forming reaction is performed under relatively mild conditions in the presence of a specific solvent, and thus the phosgenation reaction may be easily performed without an additional cooling or heating process in the phosgenation reaction, resulting in excellent economic efficiency.

### <Xylene Diisocyanate Composition>

According to one embodiment of the present invention, provided is a xylene diisocyanate composition including a xylene diisocyanate compound; and low-boiling-point compounds including isocyanomethylbenzaldehyde and isocyanomethylbenznitrile, wherein the low-boiling-point compounds are included in an amount of 1% or less with respect to the total amount of the composition. Here, the content range of the low-boiling-point compounds is GC area (%) determined by a gas chromatography (GC) analysis, and a specific measurement method thereof will be described in more detail in Experimental Example described below.

Meanwhile, when the low-boiling-point compounds are included in an amount of more than 1% in the composition, they may generate additional impurities during polyisocyanate synthesis, and interfere with the activity of the catalyst to affect the reaction rate, thereby affecting quality of a product. However, by using the xylene diisocyanate composition in which the content is controlled to the appropriate range as described above, a high-purity polyisocyanate may be prepared without such problems, and such a polyisocyanate compound may remarkably reduce the defect rate and may improve reproducibility when applied to optical articles.

The content of the above low-boiling-point compounds may be preferably 0.7% or less, 0.5% or less, or 0.3% or less with respect to the total amount of the xylene diisocyanate composition. The lower limit of the low-boiling-point compounds is 0% or more, and preferably, the low-boiling-point compounds may be included in an amount of 0.0001% or more, 0.0001% to 0.7% or 0.001 to 0.5%, and the above content range is suitable for implementing the above-described effect.

The isocyanomethylbenzaldehyde (IMBAl) may be included in an amount of 0.0001% to 0.15% with respect to the total amount of the composition, and preferably, it may be included in an amount of 0.001% to 0.15%, or 0.0003% to 0.11%.

Further, the isocyanomethylbenznitrile (IMBN) may be included in an amount of 0.0001% to 0.1% with respect to the total amount of the composition, and preferably, it may be included in an amount of 0.0003% to 0.1%, or 0.0008% to 0.06%.

According to one embodiment of the present invention, the low-boiling-point compounds may further include chloromethylbenzylisocyanate (CMBI). In this case, the chloromethylbenzylisocyanate may be included in an amount of 0.01% to 0.2% with respect to the total amount of the composition, and preferably, it may be included in an amount of 0.05% to 0.15%.

The xylene diisocyanate composition may further include additional additives to maintain storage stability, in addition to the above components.

The type of the additional additives is not particularly limited, and may further include antioxidants, thermal stabilizers, polymerization inhibitors, etc. which are commonly used in the art. The content of the additives is not particularly limited, and they may be used within an appropriate range that does not hinder the purpose of the present invention.

The xylene diisocyanate composition may be used in a wide range of fields due to its excellent physical properties, and among them, as an optical article.

The xylene diisocyanate composition may be prepared according to the preparation method described below.

### <Method of Preparing Xylene Diisocyanate Composition>

Specifically, a method of preparing a xylene diisocyanate composition according to one embodiment of the present invention includes a step of reacting an amine compound with hydrogen chloride in a solvent at 20°C to 90°C under normal pressure to obtain an amine salt compound; a step of reacting the amine salt compound with phosgene to obtain a reaction mixture including a xylene isocyanate compound; and a third step of removing the solvent and unreacted phosgene from the reaction mixture to prepare the xylene diisocyanate composition.

### (Salt-forming reaction)

First, the first step (salt-forming reaction) of reacting an amine compound with hydrogen chloride in a solvent at 20°C to 90°C under normal pressure to obtain an amine salt compound is performed.

The salt-forming reaction of obtaining the amine salt compound is performed in a specific solvent at 20°C to 90°C under normal pressure, and thus the phosgenation reaction may be easily performed without an additional cooling or heating process in the subsequent phosgenation reaction, resulting in excellent economic efficiency.

Here, normal pressure refers to the pressure in a state where a pressure-reducing equipment such as a separate vacuum pump, etc. is not applied. For example, it may correspond to approximately 760 mmHg, which is a general atmospheric condition. When performed within the above range, it is preferable that the homogeneous phase of the amine salt compound may be maintained. When the salt-forming reaction is performed by applying a pressure outside the normal pressure condition, the particle size of the amine salt compound may become non-uniform.

On the other hand, when the salt-forming reaction is performed at lower than 20°C, the particle size of the amine salt compound may become non-uniform, which may inhibit the subsequent phosgenation reaction. In addition, when it is performed at higher than 90°C, there is a problem that it is difficult to control the salt-forming reaction itself. Preferably, the salt-forming reaction may be performed at 30°C to 80°C. Meanwhile, the temperature of the salt-forming reaction may be controlled by the reaction heat resulting from the introduction of hydrogen chloride without special temperature control.

The solvent is one or more selected from the group consisting of chlorobenzene, 1,2-dichlorobenzene, 1,4-dichlorobenzene, 1,2,3-trichlorobenzene, and 1,2,4-trichlorobenzene, which are inert organic solvents that may contribute to reducing the content of by-products. In addition, these solvents may facilitate the reaction by helping to dissolve the amine salt compound due to their high polarity. Most preferably, 1,2-dichlorobenzene may be used. Meanwhile, when a solvent such as n-amyl acetate is used, the particle size of the amine salt compound may become non-uniform, which may inhibit the phosgenation reaction.

Preferably, the purity of the solvent may satisfy 99.0% or more, and accordingly, the side reaction in the phosgenation reaction may be minimized, and in particular, the content of the low-boiling-point compounds among the by-products may be controlled within the above-mentioned range, thereby preparing a high-purity xylene diisocyanate. More preferably, the purity of the solvent is 99.0% to 99.99%. The above content range is preferable in terms of implementing the above-mentioned effects.

As the purity of the amine compound satisfies 99.0% or more, side reactions in the phosgenation reaction may be minimized, and in particular, the content of the low-boiling-point compounds among the by-products is controlled within the above-mentioned range, thereby preparing a high-purity xylene diisocyanate compound. More preferably, the purity of the amine compound is 99.0% to 99.99%. The above content range is preferable in terms of implementing the above-mentioned effects.

The amine compound may be one or more selected from the group consisting of m-xylene diamine, p-xylene diamine, o-xylene diamine, and chlorides thereof (e.g., hydrochloride or carbonate, etc.). Preferably, the chloride of the amine compound may be used, which is preferable in terms of reducing the content of impurities by increasing the conversion rate into the product during the preparation of the xylene isocyanate compound described below.

More specifically, the amine compound may be obtained through the following reaction and may exhibit a purity in the above-mentioned range:
1) m-Xylene ammoxidation reaction 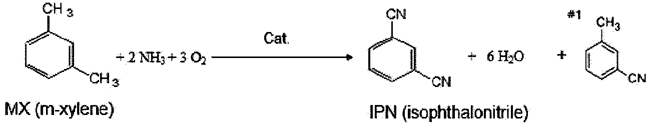
2) IPN hydrogenation reaction

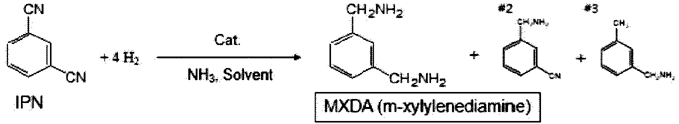

XDA is synthesized by a first step of subjecting m-xylene to ammoxidation to prepare isophthalonitrile (IPN) and then a second step of hydrogenation reaction. The impurities that may be generated during the synthesis process of the second step are indicated as #1~#3, and in addition, various other impurities may be generated, but the content of the impurities is very small (about 1% or less).

Meanwhile, the impurities may affect the content of the low-boiling-point compounds in a process of obtaining a reaction mixture including the xylene isocyanate compound described below, and in other words, as the purity of the amine compound is higher, the content of the low-boiling-point compounds may be more reduced.

The amine compound may be included in an amount of 1% by weight to 20% by weight with respect to the total amount of the solvent. When the content of the amine compound exceeds 20% by weight, a stirring process during the reaction is difficult, there is a concern about precipitation of a large amount of the amine compound, and an uneven reaction may affect the increase in the content of the low-boiling-point compounds. Preferably, the amine compound may be included in an amount of 1% by weight to 15% by weight or 5% by weight to 15% by weight.

### (Phosgenation reaction)

Next, the second step of reacting the amine salt compound with phosgene to obtain a reaction mixture including a xylene isocyanate compound is included.

By performing the salt-forming reaction under the above conditions, a homogeneous chloride is obtained, and thus the phosgenation reaction may be more easily performed.

The temperature of the reaction is not particularly limited, but the reaction may be performed at 110°C to 160°C, and more preferably, at 120°C to 140°C. When the reaction temperature is higher than 160°C, the concentration of by-products increases, and a problem of thermal decomposition of reactants and products may occur.

Preferably, the reaction may be performed by gradually increasing the temperature in the reactor to the above-mentioned range, and then by injecting phosgene, and more preferably, the reaction may be performed by controlling the temperature in the reactor to 110°C to 140°C after injecting phosgene. The reactor temperature may be preferably controlled to 120°C to 135°C.

**In** addition, according to one embodiment of the present invention, the method of injecting phosgene in the reaction of the amine salt compound with phosgene is not particularly limited. For example, the reaction may be performed by simply stirring the amine salt compound and phosgene after introducing the same into a single reactor, or by injecting phosgene into the reactor containing the amine salt compound through a mixing eductor which is a mixing nozzle. Preferably, the reaction may be performed by injecting phosgene through the mixing eductor in terms of uniformly mixing the reactants and improving the reaction efficiency, and accordingly, the reaction time may be shortened and the impurity content may be reduced, which is preferable.

The reaction time is not particularly limited, but the reaction may be performed for about 1 hour to about 4 hours after completing the injection of phosgene. Preferably, the reaction may be performed for 1 hour to 3 hours.

**In** addition, a process of reducing the temperature inside the reactor to 70°C to 80°C may be preferably further performed after completing the reaction.

### (Purification process)

Next, the third step of removing the solvent and unreacted phosgene from the reaction mixture to prepare the xylene diisocyanate composition is included.

After completing the reaction with phosgene, the solvent, the unreacted phosgene, etc. exist in the reaction mixture, which may be removed to prepare the xylene diisocyanate composition including the xylene diisocyanate compound.

Meanwhile, according to one embodiment of the present invention, the third step may further include a purification process, after removing the solvent and unreacted phosgene from the reaction mixture.

The methods of performing the removal of the solvent and phosgene and the purification process are not particularly limited, and methods commonly performed in the art may be applied. For example, unreacted phosgene and hydrogen chloride gas remaining in the reaction mixture may be removed through a nitrogen bubbling process, and the solvent may be removed through a distillation process.

The purification process may be carried out by purifying through fractional distillation under reduced pressure and thin film distillation, and the fractional distillation may be carried out with a tray distillation tower or a packed distillation tower in a plant process. The number of theoretical plates of the distillation tower is 2 or more, preferably 5 or more. Preferably, the number is 50 or less, 40 or less.

The xylene diisocyanate composition prepared according to the above-described salt-forming reaction, phosgenation reaction, and purification process includes low-boiling-point compounds of 1% or less with respect to the total amount of the composition. Here, the low-boiling-point compounds include isocyanomethylbenzaldehyde and isocyanomethylbenznitrile. The content range of the low-boiling-point compounds is GC area (%) determined by a gas chromatography (GC) analysis, and a specific measurement method thereof will be described in more detail in Experimental Example described below.

When the low-boiling-point compounds are included in an amount of more than 1%, additional impurities may be generated during subsequent synthesis of polyisocyanate, and the activity of the catalyst may be hindered, affecting the reaction rate. Accordingly, the quality of the product may be affected. However, when the xylene diisocyanate composition is used, in which the content is controlled to the appropriate range as described above, a high-purity polyisocyanate may be prepared without such problems, and this polyisocyanate compound may significantly reduce the defect rate when applied to optical articles and may improve reproducibility.

The content of the low-boiling-point compounds may be preferably 0.7% or less, 0.5% or less, or 0.3% or less with respect to the total amount of the xylene diisocyanate composition. The lower limit of the low-boiling-point compounds is 0% or more, and preferably, they may be included in an amount of 0.0001% or more, 0.0001% to 0.7%, or 0.001 to 0.5%, and the above content range is suitable for implementing the above-described effect.

The isocyanomethylbenzaldehyde (IMBAl) may be included in an amount of 0.0001% to 0.15% with respect to the total amount of the composition, and preferably, it may be included in an amount of 0.001% to 0.15%, or 0.0003% to 0.11%.

Further, the isocyanomethylbenznitrile (IMBN) may be included in an amount of 0.0001% to 0.1% with respect to the total amount of the composition, preferably, in an amount of 0.0003% to 0.1%, or 0.0008% to 0.06%.

According to one embodiment of the present invention, the low-boiling-point compounds may further include chloromethylbenzylisocyanate (CMBI). In this case, the chloromethylbenzylisocyanate may be included in an amount of 0.01% to 0.2% with respect to the total amount of the composition, preferably, in an amount of 0.05% to 0.15%.

### <Polymerizable composition>

According to one embodiment of the present invention, provided is a polymerizable composition including
the above-described xylene diisocyanate composition; and
any one or more of i) a polyfunctional thiol-based compound and ii) a polyfunctional episulfide compound.

The polymerizable composition may include the isocyanate composition, the polyfunctional thiol-based compound, and the polyfunctional episulfide-based compound in a mixed state or in a state where they are separated from each other. In other words, in the polymerizable composition, the isocyanate composition and the polyfunctional thiol-based compound or the polyfunctional episulfide-based compound may be in a mixed state where they are contacted with each other, or may be in a separated state where they are not contacted with each other.

The polyfunctional thiol-based compound may be a compound containing two or more thiol (thio, -SH) groups in the molecule, and may have an aliphatic, alicyclic, or aromatic skeleton.

The polyfunctional episulfide-based compound may be a compound containing two or more episulfides, i.e., thioepoxy groups in the molecule, and may have an aliphatic, alicyclic, or aromatic skeleton.

According to one embodiment, the polyfunctional thiol-based compound may include one or more selected from 4,8-bis(mercaptomethyl)-3,6,9-trithiaundecan-1,11-dithiol, 4,7-bis(mercaptomethyl)-3,6,9-trithiaundecan-1,11-dithiol, 5,7-bis(mercaptomethyl)-3,6,9-trithiaundecan-1,11-dithiol, bis(2-mercaptoethyl)sulfide, 4-mercaptomethyl-3,6-dithiaoctane-1,8-dithiol, 2,3-bis(2-mercaptoethylthio)propane-1-thiol, 2,2-bis(mercaptomethyl)propane-1,3-dithiol, 2-(2-mercaptoethylthio)propane-1,3-dithiol, 2-(2,3-bis(2-mercaptoethylthio)propylthio)ethanethiol, bis(2,3-dimercaptopropanyl)sulfide, bis(2,3-dimercaptopropanyl)disulfide, 1,2-bis(2-(2-mercaptoethylthio)-3-mercaptopropylthio)ethane, bis(2-(2-mercaptoethylthio)-3-mercaptopropyl)disulfide, 2-(2-mercaptoethylthio)-2-mercapto-3-[3-mercapto-2-(2-mercaptoethylthio)-propylthio]propylthio-propane-1-thiol, 2-(2-mercaptoethylthio)-3-mercapto-3-[3-mercapto-2-(2-mercaptoethylthio)-propylthio]propylthio-propane-1-thiol, 2-(2-mercaptoethylthio)-3-(2-(2-[3-mercapto-2-(2-mercaptoethylthio)-propylthio]ethylthio)ethylthio)-propane-1-thiol, (4R,11S)-4,11-bis(mercaptomethyl)-3,6,9,12-tetrathiatetradecane-1,14-dithiol, (S)-3-((R-2,3-dimercaptopropyl)thio)propane-1,2-dithiol, 4,14-bis(mercaptomethyl)-3,6,9,12,15-pentathiaheptadecane-1,17-dithiol, (S)-3-((R-3-mercapto-2-((2-mercaptoethyl)thio)propyl)thio)propyl)thio)-2-((2-mercaptoethyl)thio)propane-1-thiol, 3,3'-dithiobis(propane-1,2-dithiol), (7R,11S)-7,11-bis(mercaptomethyl)-3,6,9,12,15-pentathiaheptadecane-1,17-dithiol, (7R,12S)-7,12-bis(mercaptomethyl)-3,6,9,10,13,16-hexathiaoctadecane-1,18-dithiol, 2-(2-mercaptoethylthio)-3-[4-(1-{4-[3-mercapto-2-(2-mercaptoethylthio)-propoxy]-phenyl}-1-methylethyl)-phenoxy]-propane-1-thiol, 2,2-bis-(3-mercapto-propionyloxymethyl)-butyl ester, pentaerythritol tetrakis(3-mercaptopropionate), pentaerythritol tetrakis(2-mercaptoacetate), bispentaerythritol-ether-hexakis(3-mercaptopropionate), trimethylolpropane tris(2-mercaptopropionate), trimethylolpropane tris(3-mercaptopropionate), glycerol trimercaptopropionate, 1,1,3,3-tetrakis(mercaptomethylthio)propane, 1,1,2,2-tetrakis(mercaptomethylthio)ethane, 4,6-bis(mercaptomethylthio)-1,3-dithiane, 2-(2,2-bis(mercaptomethylthio)ethyl)-1,3-dithiane, and 2,5-bismercaptomethyl-1,4-dithiane.

According to one embodiment, the polyfunctional episulfide-based compound may include one or more selected from bis(β-epithiopropylthio)methane, 1,2-bis(β-epithiopropylthio)ethane, 1,3-bis(β-epithiopropylthio)propane, 1,2-bis(β-epithiopropylthio)propane, 1-(β-epithiopropylthio)-2-(β-epithiopropylthiomethyl)propane, 1,4-bis(β-epithiopropylthio)butane, 1,3-bis(β-epithiopropylthio)butane, 1-(β-epithiopropylthio)-3-(β-epithiopropylthiomethyl)butane, 1,5-bis(β-epithiopropylthio)pentane, 1-(β-epithiopropylthio)-4-(β-epithiopropylthiomethyl)pentane, 1,6-bis(β-epithiopropylthio)hexane, 1-(β-epithiopropylthio)-5-(β-epithiopropylthiomethyl)hexane, 1-(β-epithiopropylthio)-2-[(2-β-epithiopropylthioethyl)thio]ethane, 1-(β-epithiopropylthio)-2-[[2-(2-β-epithiopropylthioethyl)thioethyl]thio]ethane, tetrakis(β-epithiopropylthiomethyl)methane, 1,1,1-tris(β-epithiopropylthiomethyl)propane, 1,5-bis(β-epithiopropylthio)-2-(β-epithiopropylthiomethyl)-3-thiapentane, 1,5-bis(β-epithiopropylthio)-2,4-bis(β-epithiopropylthiomethyl)-3-thiapentane, 1-(β-epithiopropylthio)-2,2-bis(β-epithiopropylthiomethyl)-4-thiahexane, 1,5,6-tris(β-epithiopropylthio)-4-(β-epithiopropylthiomethyl)-3-thiahexane, 1,8-bis(β-epithiopropylthio)-4-(β-epithiopropylthiomethyl)-3,6-dithiaoctane, 1,8-bis(β-epithiopropylthio)-4,5-bis(β-epithiopropylthiomethyl)-3,6-dithiaoctane, 1,8-bis(β-epithiopropylthio)-4,4-bis(β-epithiopropylthiomethyl)-3,6-dithiaoctane, 1,8-bis(β-epithiopropylthio)-2,4,5-tris(β-epithiopropylthiomethyl)-3,6-dithiaoctane, 1,8-bis(β-epithiopropylthio)-2,5-bis(β-epithiopropylthiomethyl)-3,6-dithiaoctane, 1,9-bis(β-epithiopropylthio)-5-(β-epithiopropylthiomethyl)-5-[(2-β-epithiopropylthioethyl)thiomethyl]-3,7-dithianonane, 1,10-bis(β-epithiopropylthio)-5,6-bis[(2-β-epithiopropylthioethyl)thio]-3,6,9-trithiadecane, 1,11-bis(β-epithiopropylthio)-4,8-bis(β-epithiopropylthiomethyl)-3,6,9-trithiaundecane, 1,11-bis(β-epithiopropylthio)-5,7-bis(β-epithiopropylthiomethyl)-3,6,9-trithiaundecane, 1,11-bis(β-epithiopropylthio)-5,7-[(2-β-epithiopropylthioethyl)thiomethyl]-3,6,9-trithiaundecane, 1,11-bis(β-epithiopropylthio)-4,7-bis(β-epithiopropylthiomethyl)-3,6,9-trithiaundecane, 1,3-bis(β-epithiopropylthio)cyclohexane, 1,4-bis(β-epithiopropylthio)cyclohexane, 1,3-bis(β-epithiopropylthiomethyl)cyclohexane, 1,4-bis(β-epithiopropylthiomethyl)cyclohexane, bis[4-(β-epithiopropylthio)cyclohexyl]methane, 2,2-bis[4-(β-epithiopropylthio)cyclohexyl]propane, bis[4-(β-epithiopropylthio)cyclohexyl] sulfide, 2,5-bis(β-epithiopropylthiomethyl)-1,4-dithiane, 2,5-bis(β-epithiopropylthioethylthiomethyl)-1,4-dithiane, 1,3-bis(β-epithiopropylthio)benzene, 1,4-bis(β-epithiopropylthio)benzene, 1,3-bis(β-epithiopropylthiomethyl)benzene, 1,4-bis(β-epithiopropylthiomethyl)benzene, bis[4-(β-epithiopropylthio)phenyl]methane, 2,2-bis[4-(β-epithiopropylthio)phenyl]propane, bis[4-(β-epithiopropylthio)phenyl] sulfide, bis[4-(β-epithiopropylthio)phenyl] sulfone, and 4,4'-bis(β-epithiopropylthio)biphenyl.

In the polymerizable composition, a molar ratio of (thio group + episulfide group) to isocyanate group may be about 0.5 to about 1.5, or about 0.8 to about 1.2, or about 0.9 to about 1.1, but the present invention is not necessarily limited thereto.

In addition, the polymerizable composition may further include an appropriate amount of additives such as a release agent, a thermal stabilizer, an ultraviolet stabilizer, a pigment, a urethane reaction catalyst, etc.

The release agent is a type of surfactant component, and may be exemplified by a fluorine-based nonionic surfactant containing a perfluoroalkyl group; a silicone-based nonionic surfactant containing a dimethylpolysiloxane group; a quaternary ammonium salt such as a trimethyl cetyl ammonium salt, a trimethyl stearyl, a dimethylethyl cetyl ammonium salt, a triethyl dodecyl ammonium salt, a trioctylmethyl ammonium salt, and a diethylcyclohexadodecyl ammonium salt, etc.

The thermal stabilizer may be, for example, a metal fatty acid salt-based compound, a phosphorus-based compound, a lead-based compound, an organotin-based compound, etc. These compounds may be used alone or in combination of two or more thereof.

The ultraviolet stabilizer may be, for example, a benzophenone-based compound, a benzotriazole-based compound, a salicylate-based compound, a cyanoacrylate-based compound, an oxanilide-based compound, etc.

The pigment may be, for example, a fluorescent whitening agent, a fluorescent pigment, an inorganic pigment, etc.

The urethane reaction catalyst may be, for example, a dialkyltin halide-based compound such as dibutyltin dichloride, dimethyltin dichloride, etc.; a dialkyltin dicarboxylate-based compound such as dimethyltin diacetate, dibutyltin dioctanoate, dibutyltin dilaurate, etc.; a dialkyltin dialkoxide-based compound such as dibutyltin dibutoxide, dioctyltin dibutoxide, etc.; a dialkyltin dithioalkoxide-based compound such as dibutyltin di(thiobutoxide), etc.; a dialkyltin oxide compound such as di(2-ethylhexyl)tin oxide, dioctyltin oxide, bis(butoxydibutyltin) oxide, etc.; a dialkyltin sulfide-based compound, etc., which may be used alone or in combination of two or more thereof.

### <Optical Article>

Further, according to one embodiment of the present invention, provided is an optical article including a polythiourethane polymer which is prepared from the polymerizable composition.

More preferably, the optical article may be an optical lens, for example, a spectacle lens, a camera lens, a plastic lens, a prism, etc.

### <Method of Preparing Polyisocyanate Composition>

According to another embodiment of the present invention, provided is a method of preparing a polyisocyanate composition to which the method of preparing the xylene diisocyanate composition is applied.

Specifically, the method of preparing a polyisocyanate composition according to one embodiment of the present invention includes
a first step of reacting an amine compound with hydrogen chloride in a solvent at 20°C to 90°C under normal pressure to obtain an amine salt compound;
a second step of reacting the amine salt compound with phosgene to obtain a reaction mixture including a xylene diisocyanate compound;
a third step of removing the solvent and unreacted phosgene from the reaction mixture to prepare the xylene diisocyanate composition; and
a fourth step of polymerizing the xylene diisocyanate composition and polyhydric alcohol to synthesize the polyisocyanate compound.

The above-described method of preparing the xylene diisocyanate composition may be applied equally to all the first, second, and third steps.

Accordingly, the solvent is one or more selected from the group consisting of chlorobenzene, 1,2-dichlorobenzene, 1,4-dichlorobenzene, 1,2,3-trichlorobenzene, and 1,2,4-trichlorobenzene, and the purity of the amine compound is 99.0% or higher, and the xylene diisocyanate composition includes low-boiling-point compounds of 1% or less with respect to the total amount of the composition. Here, the low-boiling-point compounds include isocyanomethylbenzaldehyde and isocyanomethylbenznitrile.

Next, the fourth step of mixing and polymerizing the xylene diisocyanate composition and polyhydric alcohol to synthesize the polyisocyanate compound is included.

The isocyanate compound included in the composition has high purity, and since the low-boiling-point compounds in the composition satisfies 1% or less, the generation of by-products in the polymerization step is remarkably reduced.

Preferably, the polyhydric alcohol is a compound containing two or more hydroxyl groups in one molecule, and specifically, it may be a compound having two or more, or three or more, and eight or less, or four or less hydroxyl groups in the molecule.

Specific examples may include dihydric alcohols such as ethylene glycol, diethylene glycol, triethylene glycol, 1,2-propanediol, 1,3-propanediol, 1,2-butanediol, 1,4-butanediol, 2-methyl-2,3-butanediol, 1,6-hexanediol, 1,2-hexanediol, etc.; trihydric alcohols such as glycerol, trimethylolethane, trimethylolpropane (TMP), etc.; tetrahydric alcohols such as diglycerin, ditrimethylolpropane, pentaerythritol, dipentaerythritol, etc.; pentahydric alcohols such as L-arabinitol, ribitol, xylitol, etc.; hexahydric alcohols such as D-glucitol, D-mannitol, galactitol, etc.; heptahydric alcohols such as trehalose, etc.; octahydric alcohols such as sucrose, maltose, etc., or low molecular weight polyols, and among them, diethylene glycol, glycerol, trimethylolethane, trimethylolpropane, or mixtures thereof may be used. More specifically, it may be preferable that a trihydric alcohol such as glycerol, trimethylolpropane, or trimethylolethane is used alone, or a mixture of the trihydric alcohol and another polyhydric alcohol is used.

Meanwhile, the polymerization reaction is performed by a urethane reaction (or addition polymerization reaction) between the isocyanate compound and the hydroxyl group in the polyhydric alcohol.

Accordingly, it may be desirable to appropriately determine the amount of use of the polyhydric alcohol in consideration of the urethane reaction with the isocyanate compound, physical properties to be achieved in the prepared polyisocyanate, such as viscosity, etc., and the use of the polymer, etc. Specifically, the polyhydric alcohol may be introduced in such an amount that a molar ratio of the hydroxyl group in the polyhydric alcohol is 0.05 or more, or 0.15 or more, and 1 or less, or 0.8 or less with respect to 1 mole of the isocyanate group of the isocyanate compound. When the molar ratio of the hydroxyl group to the isocyanate group is less than 0.05 outside the above molar ratio range, the viscosity of the prepared polymer may decrease due to the excess isocyanate group, and as a result, there is a concern that processability may decrease. Further, when the molar ratio of hydroxyl groups to the isocyanate group is more than 1, there is a concern that the effect of preventing discoloration may be reduced due to excess hydroxyl groups.

The polymerization reaction may be performed under atmospheric pressure and an inert gas atmosphere such as nitrogen or argon, etc.

The polymerization reaction is preferably performed at a temperature range of 40°C or higher, or 60°C or higher, and 100°C or lower, or 80°C or lower, because it is possible to easily control the reaction rate without concern about discoloration and also to increase the reaction efficiency.

The polymerization reaction may be performed under non-catalytic conditions, or may be performed in the presence of a catalyst that usually promotes the urethane reaction, such as a tin-based or amine-based catalyst, etc. When performed in the presence of the catalyst, the catalyst may be further introduced when polyhydric alcohol is added to the monomer composition.

The progress of the polymerization reaction may be estimated by measuring the concentration of isocyanate groups in the polymerization reaction product by an n-dibutyl amine method using a potentiometric titrator, or by measuring a refractive index. **In** the present invention, the polymerization reaction is performed until the concentration of the isocyanate groups in the polymerization reaction product reaches the calculated value of the isocyanate groups remaining after reacting with polyhydric alcohol.

As a result of the polymerization reaction described above, polyisocyanate is prepared.

The polyisocyanate prepared as described above may be prepared into polyurethane through a reaction with polyol, and precise control of the physical properties of the polyurethane product is possible by using the polyisocyanate according to the present invention.

The polyisocyanate specifically includes a urethane bond formed by reacting a part or all of the isocyanate group of the isocyanate compound in the reaction mixture obtained in the aforementioned phosgenation reaction with the hydroxyl group of the polyhydric alcohol.

In the present invention, the high-purity xylene diisocyanate compound is included in the reaction mixture, and particularly, the content of the low-boiling-point compounds is 1% by weight or less, and thus high-purity polyisocyanate may be prepared in a high yield, and a product without discoloration and white turbidity may be prepared. More preferably, the content may be 0.7% or less, 0.5% or less, or 0.3% or less with respect to the total amount of the composition. The lower limit of the low-boiling-point compounds is 0% or more, and preferably, they may be included in an amount of 0.0001% or more, 0.0001% to 0.7%, or 0.001% to 0.5%. The content range of the low-boiling-point compounds is GC area (%) determined by a gas chromatography (GC) analysis, and a specific measurement method thereof will be described in more detail in Experimental Example described below.

In the resulting product obtained as a result of the polymerization reaction, a stabilizer and unreacted diisocyanate that did not participate in the polymerization reaction may exist, in addition to the polyisocyanate.

Therefore, the method of preparing the polyisocyanate composition according to one embodiment of the present invention may optionally further include a step of removing unreacted diisocyanate by purifying the resulting product obtained as a result of completing the polymerization reaction.

The purification process may be performed by a common purification method such as distillation or solvent extraction, etc., and in the present invention, it may be performed by a distillation purification method such as a thin film distillation method, in terms of excellent efficiency in removing unreacted polyisocyanate.

The pressure and temperature during the distillation purification process may be appropriately controlled according to the composition of the polyisocyanate composition, the distillation device, etc. In the present invention, the distillation purification process may be performed under a pressure of 0.001 kPa or more, 1 kPa or less, or 0.5 kPa or less.

In addition, the distillation purification process may be performed at a temperature of 70°C or higher, or 90°C or higher, and 200°C or lower, or 180°C or lower. When the temperature is lower than 70°C, there is a concern that the distillation purification efficiency may reduce, and when the temperature is higher than 200°C, there is a concern that the polyisocyanate may be deteriorated due to the high-temperature heat.

The above distillation purification process may reduce the content of unreacted diisocyanate in the polyisocyanate composition, and as the content is lower, the stability of the composition more increases, which is preferable.

### <Polyisocyanate Composition>

According to still another embodiment of the present invention, provided is a composition prepared according to the method of preparing the polyisocyanate composition.

The polyisocyanate composition may further include a diluent solvent, and accordingly, it exhibits appropriate coating properties, making it easy to apply the same to a product.

Preferably, ethyl acetate may be used as the diluent solvent, and for example, it may be included such that the solid content of the isocyanate group (NCO%) in the composition is 75 wt%. As the content of the isocyanate group is within the above range, an appropriate crosslinking density may be exhibited, and when the polymer composition is applied, it may exhibit excellent characteristics of forming a coating film.

Meanwhile, in the present invention, NCO% may be obtained by neutralizing the isocyanate group with an excess of 2 N amine and then performing a back titration using 1 N hydrochloric acid.

The content of remaining unreacted diisocyanate based on the total weight of solids in the polyisocyanate composition is 1% by weight or less, or 0.5% by weight or less, or 0.3% by weight or less, and thus the content of unreacted diisocyanate is greatly reduced, as compared to the prior art, thereby exhibiting superior stability.

The polyisocyanate composition may further include additives such as an internal release agent, an ultraviolet absorber, a polymerization initiator, a thermal stabilizer, a color correction agent, a chain extender, a crosslinking agent, a light stabilizer, a filler, etc., as needed, and the content thereof may be appropriately determined within a range that does not inhibit the coloring and discoloration-suppression properties of the polymer composition.

The polyisocyanate composition may be used in a wide range of fields due to its excellent physical properties, and among them, due to its excellent stickiness/adhesiveness, it may be used as a sticking agent or adhesive agent.

Hereinafter, the actions and effects of the present invention will be described in more detail with reference to specific exemplary embodiments of the present invention. However, these exemplary embodiments are provided only for illustrating the present invention, but the scope of the present invention is not limited thereby.

### [Preparation Example - Preparation of xylene diisocyanate composition]

### Preparation Example 1

In a reactor, 437 kg of 1,2-dichlorobenzene with a purity of 99.83% and 45.5 kg (10.4 wt%) of meta-xylenediamine (m-XDA) with a purity of 99.5% were stirred at room temperature (about 24°C) and atmospheric pressure (1 atm) while injecting 27 kg of anhydrous hydrochloric acid for 3 hours. As the temperature increased while injecting anhydrous hydrochloric acid, a salt-forming reaction was initiated, and when the reactor was maintained at atmospheric pressure (1 atm) and about 80°C for about 4 hours, the salt-forming reaction proceeded to obtain an amine salt compound.

Next, 40 kg of phosgene was initially injected into the reactant containing the above amine salt compound, and the temperature was raised to 120°C. In addition, 300 kg of phosgene was slowly injected into the reactor, and then the reactor temperature was maintained at 120°C to 135°C. 20 hours was taken from the time of phosgene injection to the end of the reaction, and after the solution became transparent, the inside of the reactor was cooled to 80°C and cooled by blowing nitrogen. A xylene diisocyanate composition from which phosgene had been removed was obtained.

### Preparation Example 2

The same amounts as in Preparation Example 1 were used, but upon injecting phosgene, an educator nozzle was installed at the injection end, and phosgene was injected at 80°C, where the salt-forming reaction was completed, and the temperature was raised to 120°C. The internal temperature was controlled by adjusting the flow rate of phosgene. The total reaction time was 15 hours.

### Preparation Example 3

The same amounts as in Preparation Example 2 were used, but upon injecting phosgene, an educator nozzle was installed at the injection end, and when the salt-forming reaction was completed, the temperature was raised, phosgene was injected at 120°C, and the reactor temperature was maintained at 120°C to 135°C. The total reaction time was 12 hours.

### [Experimental Example 1: Analysis of Preparation Conditions of Isocyanate Compound]

For the xylene diisocyanate composition including each of the isocyanate compounds prepared in Preparation Examples, gas chromatography (GC) analysis was performed, and GC area (%) for m-XDI, excluding the solvent, is shown as the m-XDI purity in Table 1.

### <GC analysis conditions>

The phosgenation reaction product was analyzed using GC. The GC used for the analysis was HP-6890, and detection was performed with FID. The used column was DB-17 (30 m * 0.25 mm * 0.5 µm), the carrier gas was nitrogen (1.0 mL/min), the injection amount was 1 µl, and the oven temperature was 80°C -> 5°C/min -> 160°C(8 min) -> 20°C/min -> 280°C(18 min).
Split ratio: Pulsed splitless method
IMBAl detection method: SIM (monitoring ion: m/z 161, 132)
CMBI detection method: SIM (monitoring ion: m/z 181, 146)
IMBAl detection method: SIM (monitoring ion: m/z 158, 116)

**[Table 1]**

| Section | Preparation Example 1 | Preparation Example 2 | Preparation Example 3 |
|---|---|---|---|
| Phosgenation reaction time (hr) | 20 | 15 | 12 |
| m-XDI purity (%) | 98.3 | 98.5 | 98.9 |

As shown in Table 1, it was confirmed that the reaction uniformity varied depending on the phosgene injection method in the phosgenation reaction, and thus there was a difference in the reaction efficiency. In particular, it was confirmed that when the phosgene mixing eductor was used, as in Preparation Examples 2 and 3, phosgene and the amine salt compound were uniformly mixed, and thus the reaction time was shortened and the purity of m-XDI was increased.

### [Examples and Comparative Examples - Preparation of Polyisocyanate Composition]

### Example 1

### Preparation of isocyanate compound

A reaction mixture including an isocyanate compound was prepared according to Preparation Example 3 (m-XDA: 10.4 wt%), and the solvent was removed through reduced pressure distillation from the reaction solution from which phosgene had been removed. The product was purified through fractional distillation under reduced pressure and thin-film distillation to obtain a xylene diisocyanate composition including the isocyanate compound.

### Preparation of polyisocyanate composition

Under a nitrogen atmosphere, 300 g of the prepared xylene diisocyanate composition including the isocyanate compound (meta-xylene diisocyanate) was put in a flask. The temperature of the flask was raised to 70°C and then maintained, and 26.7 g of trimethylolpropane (TMP) as a polyhydric alcohol was added dropwise. Thereafter, the reaction temperature was maintained at 70°C until the concentration of the isocyanate group in the flask reached the calculated value of 33%.

After completing the reaction, the resulting reaction product was purified using a thin film evaporator (TFE) to separate unreacted XDI, and diluted to a solid content of 75% by weight using ethyl acetate, thereby obtaining a polyisocyanate composition.

### Example 2

A polyisocyanate composition was obtained in the same manner as Example 1, except that the amount of m-XDA was changed to 8 wt%.

### Example 3

A polyisocyanate composition was obtained in the same manner as Example 1, except that the amount of m-XDA was changed to 5 wt%.

### Example 4

An isocyanate compound and a polyisocyanate composition were obtained in the same manner as Example 1, except that m-XDA having a purity of 99.83% in Example 1 was purified to 99.97%, which was used.

### Example 5

A polyisocyanate composition was obtained in the same manner as Example 4, except that the amount of m-XDA was changed to 8 wt%.

### Example 6

A polyisocyanate composition was obtained in the same manner as Example 4, except that the amount of m-XDA was changed to 5 wt%.

### Comparative Example 1 (Direct phosgenation reaction)

362 ml of 1,2-dichlorobenzene and 42.7 ml of phosgene were charged into a flask, the temperature was cooled to -10°C to -15°C, and 31.5 ml of m-XDA (8 vol%) was slowly introduced. After completing the introduction of the amine compound, the reactor temperature was heated to 130°C, and until the reaction solution became transparent, the reactor temperature was maintained at 125°C to 135°C to carry out a direct phosgenation reaction. After the reaction solution became transparent, nitrogen was introduced into the reactor and the temperature was reduced to 80°C. Thereafter, the solvent was removed through vacuum distillation, and the product was purified through fractional distillation under reduced pressure to obtain a composition including an isocyanate compound and a polyisocyanate composition.

### Comparative Example 2 (Control of purity of reactant)

A polyisocyanate composition was obtained in the same manner as Example 1, except that m-XDA with a purity of 98.0% was used.

### Comparative Example 3 (Control of solvent)

A polyisocyanate composition was obtained in the same manner as Example 1, except that n-amyl acetate was used as a solvent.

### Comparative Example 4 (Control of process conditions of salt-forming reaction - pressure)

A polyisocyanate composition was obtained in the same manner as Example 1, except that during the salt-forming reaction, the pressure was changed to 0.5 kgf/cm² ~ 1.02 kgf/cm² at room temperature.

### Comparative Example 5 (Control of process conditions of salt-forming reaction - temperature)

In Example 1, the temperature of the reactor was heated to about 120°C during the salt-forming reaction to proceed the reaction. As a result, the evaporation amount of XDA.HCl increased, and thus a large amount of XDA.HCl salt was deposited in the condenser and line of the reactor, and thus the reaction product could not be obtained. Accordingly, the isocyanate composition could not be obtained.

### Comparative Example 6 (Control of process conditions of salt-forming reaction - temperature)

A polyisocyanate composition was obtained in the same manner as Example 1, except that during the salt-forming reaction, the reactor temperature was cooled to about 15±1°C.

### [Experimental Example 2: Evaluation of isocyanate compound]

For a reaction mixture including each of the isocyanate compounds prepared in Examples and Comparative Examples, gas chromatography (GC) analysis was performed, and the results are shown as GC area (%) in Table 2.

### <GC analysis conditions>

The GC used for the analysis was HP-6890, and detection was performed with FID. The column used was DB-17 (30 m * 0.25 mm * 0.5 µm), the carrier gas was nitrogen (1.0 mL/min), the injection amount was 1 µl, and the oven temperature was 80°C -> 5°C/min -> 160°C(8 min) - > 20°C/min -> 280°C(18 min).

### <Content (%) of unreacted XDA after salt forming>

After completing the salt-forming reaction, a sample was collected and the content of remaining XDA was determined as GC area (%).

### [Experimental Example 3: Analysis of polyisocyanate composition]

For the polyisocyanate compositions prepared in Examples and Comparative Examples, color, NCO content, remaining XDI content, and white turbidity were measured according to methods described below, and the results are shown in Table 2.

For the polyisocyanate composition samples prepared above, color was evaluated according to the APHA method at 25°C.

### [Experimental Example 4: Evaluation of optical article]

20.8 g of the reaction mixture containing each of the isocyanate compounds prepared in Examples and Comparative Examples, 0.04 g of zelec UN (manufactured by Stepan), and 0.04 g of biosorb 583 (manufactured by Sakai Chemical industry Co., Ltd) were stirred in a flask at room temperature for about 20 minutes. Next, 0.002 g of dibutyltin chloride was further added, stirred for 10 minutes, and 19.2 g of 2,3-bis(2- sulfanyl ethyl sulfanyl)propane-1-thiol was added to the mixture, degassed at 5 mbar, and stirred for 1 hour to prepare a liquid mixture.

This liquid mixture was filtered through a 1 µm PTFE filter and injected into a mold consisting of a glass mold and tape. This mold was placed in an oven, and the temperature was gradually raised from 10°C to 120°C, and a polymerization reaction was performed for 20 hours. After completing the polymerization, the mold was taken out from the oven and released to obtain a plastic lens. The obtained plastic lens was annealed at 120°C for 6 hours to manufacture a final optical lens sample.

For the manufactured optical lens, the degree of occurrence of white turbidity (transparency) was evaluated with the naked eye under various light source conditions according to the evaluation criteria below, and the results are shown in Table 2.

### <Evaluation criteria>

### C (Clear): Transparent under both fluorescent and zirconium lamps

S.H (Slightly lamp Haze): Transparent under fluorescent lamp, but some turbidity observed under zirconium lamp
L.H (Lamp Haze): Transparent under fluorescent lamp, but turbidity observed under zirconium lamp
V.H (Visual Haze): Turbidity observed under both fluorescent and zirconium lamps
Y.I: Yellowing observed in the lens

**[Table 2]**

| Section | m-XDA purity (%) | Unreacted XDA content after salt forming (%) | Mean particle size D[4,3] after salt forming (µm) | IMBAl content (%) | CMBI content (%) | IMBN content (%) | IMBAl+C MBI+IMB N content (%) | Evaluation of optical lens | APHA after polyisocya nate preparation |
|---|---|---|---|---|---|---|---|---|---|
| Example1 | 99.83 | 0.18 | 6 | 0.11 | 0.13 | 0.06 | 0.30 | C | 14 |
| Example2 | 99.83 | 0.07 | 5 | 0.09 | 0.10 | 0.04 | 0.23 | C | 9 |
| Example3 | 99.83 | 0.19 | 4 | 0.08 | 0.06 | 0.02 | 0.16 | C | 6 |
| Example4 | 99.97 | 0.10 | 6 | 0.03 | 0.11 | 0.004 | 0.144 | C | 7 |
| Example5 | 99.98 | 0.08 | 6 | 0.009 | 0.067 | 0.0009 | 0.0769 | C | 6 |
| Example6 | 99.98 | 0.07 | 5 | 0.003 | 0.05 | 0.0008 | 0.0538 | C | 6 |
| Comparative Example1 | 99.83 | - | - | 0.23 | 2.12 | 0.11 | 2.46 | V.HY.I | 106 |
| Comparative Example2 | 98 | 0.25 | - | 0.59 | 0.8 | 0.84 | 2.23 | L.HY.I | 40 |
| Comparative Example3 | 99.83 | 0.88 | 54 | 0.21 | 2.00 | 0.01 | 2.22 | V.HY.I | - |
| Comparative Example4 | 99.83 | 0.29 | 167 | 0.22 | 0.53 | 0.14 | 0.89 | S.H | 36 |
| Comparative Example5 | 99.83 | - | - | - | - | - | - | - | - |
| Comparative Example6 | 99.83 | 0.49 | 38 | 0.25 | 0.67 | 0.11 | 1.03 | S.H | 31 |

As shown in Table 1, in Examples, high-purity xylene diisocyanate compounds could be prepared in high yields through a simple preparation process of controlling the purity of the reaction composition, and at the same time, controlling the content range of particular low-boiling-point compounds among the by-products. In addition, the high-purity xylene diisocyanate could be prepared by controlling the salt-forming reaction conditions of the amine compound to an appropriate range and the process efficiency in the subsequent phosgenation reaction was increased. In the optical lenses using the same, excellent transparency was achieved, as compared to Comparative Examples. It was also confirmed that the color value according to the APHA method was low even after preparing the polyisocyanate composition using the xylene diisocyanate prepared according to the above method.

In Comparative Examples, it was confirmed that as the process conditions changed, the amount of impurities increased, or the particle size became uneven after the salt-forming reaction, which inhibited the phosgenation reaction, and accordingly, the quality, such as transparency and color, which are considered when applied to products, deteriorated, as compared to Examples.

## Claims

1. A xylene diisocyanate composition comprising:
a xylene diisocyanate compound; and
low-boiling-point compounds including isocyanomethylbenzaldehyde and isocyanomethylbenznitrile,
wherein the low-boiling-point compounds are included in an amount of 1% or less with respect to the total amount of the composition.

2. The xylene diisocyanate composition of claim 1, wherein the low-boiling-point compounds are included in an amount of 0.0001% to 0.7% with respect to the total amount of the composition.

3. The xylene diisocyanate composition of claim 1, wherein the isocyanomethylbenzaldehyde is included in an amount of 0.0001% to 0.15% with respect to the total amount of the composition.

4. The xylene diisocyanate composition of claim 1, wherein the isocyanomethylbenznitrile is included in an amount of 0.0001% to 0.1% with respect to the total amount of the composition.

5. The xylene diisocyanate composition of claim 1, wherein the low-boiling-point compounds further include chloromethylbenzylisocyanate.

6. The xylene diisocyanate composition of claim 5, wherein the chloromethylbenzylisocyanate is included in an amount of 0.01% to 0.2% with respect to the total amount of the composition.

7. A method of preparing a xylene diisocyanate composition, the method comprising:
a first step of reacting an amine compound with hydrogen chloride in a solvent at 20°C to 90°C under normal pressure to obtain an amine salt compound;
a second step of reacting the amine salt compound with phosgene to obtain a reaction mixture including a xylene isocyanate compound; and
a third step of removing the solvent and unreacted phosgene from the reaction mixture to prepare the xylene diisocyanate composition,
wherein the solvent is one or more selected from the group consisting of chlorobenzene, 1,2-dichlorobenzene, 1,4-dichlorobenzene, 1,2,3-trichlorobenzene, and 1,2,4-trichlorobenzene,
a purity of the amine compound is 99.0% or more, and
the xylene diisocyanate composition includes low-boiling-point compounds of 1% or less with respect to the total amount of the composition, and the low-boiling-point compounds include isocyanomethylbenzaldehyde and isocyanomethylbenznitrile.

8. The method of claim 7, wherein the low-boiling-point compounds are included in an amount of 0.0001% to 0.7% with respect to the total amount of the composition.

9. The method of claim 7, wherein the low-boiling-point compounds further include chloromethylbenzylisocyanate.

10. The method of claim 7, wherein the amine compound is one or more selected from the group consisting of m-xylene diamine, p-xylene diamine, o-xylene diamine, and chlorides thereof.

11. The method of claim 7, wherein the amine compound is included in an amount of 1% by weight to 20% by weight with respect to the total amount of the solvent.

12. The method of claim 7, wherein the solvent is 1,2-dichlorobenzene.

13. The method of claim 7, wherein the second step is performed at 110°C to 160°C.

14. The method of claim 7, wherein the second step is performed by introducing the phosgene compound after heating the amine compound in the solvent at a temperature of 110°C to 140°C.

15. The method of claim 7, wherein the second step is performed by spraying phosgene into a reactor containing the amine salt compound through a mixing eductor.

16. The method of claim 7, wherein the third step further includes a purification process after removing the solvent and unreacted phosgene from the reaction mixture.

17. A method of preparing a polyisocyanate composition, the method comprising:
a first step of reacting an amine compound with hydrogen chloride in a solvent at 20°C to 90°C under normal pressure to obtain an amine salt compound;
a second step of reacting the amine salt compound with phosgene to obtain a reaction mixture including a xylene diisocyanate compound;
a third step of removing the solvent and unreacted phosgene from the reaction mixture to prepare the xylene diisocyanate composition; and
a fourth step of polymerizing the xylene diisocyanate composition and polyhydric alcohol to synthesize the polyisocyanate compound,
wherein the solvent is one or more selected from the group consisting of chlorobenzene, 1,2-dichlorobenzene, 1,4-dichlorobenzene, 1,2,3-trichlorobenzene, and 1,2,4-trichlorobenzene,
a purity of the amine compound is 99.0% or more, and
the xylene diisocyanate composition includes low-boiling-point compounds of 1% or less with respect to the total amount of the composition, and the low-boiling-point compounds include isocyanomethylbenzaldehyde and isocyanomethylbenznitrile.

18. The method of claim 17, wherein the low-boiling-point compounds are included in an amount of 0.0001% to 0.7% with respect to the total amount of the composition.

19. The method of claim 17, wherein the low-boiling-point compounds further include chloromethylbenzylisocyanate.

20. The method of claim 17, wherein the polyhydric alcohol is a trihydric alcohol or a mixture of the trihydric alcohol and another polyhydric alcohol.

21. The method of claim 17, wherein the polyhydric alcohol includes diethylene glycol, glycerol, trimethylolethane, trimethylolpropane, or mixtures thereof.

22. The method of claim 17, wherein the second step is performed under an inert gas atmosphere at a temperature range of 40°C to 100°C.

23. The method of claim 17, wherein the third step further includes a purification process after removing the solvent and unreacted phosgene from the reaction mixture.

24. A polymerizable composition comprising:
the xylylene diisocyanate composition of any one of claims 1 to 6; and
any one or more of i) polyfunctional thiol-based compounds and ii) polyfunctional episulfide-based compounds.

25. An optical article comprising a polythiourethane polymer which is prepared from the polymerizable composition of claim 24.

26. The optical article of claim 25, wherein the optical article is an optical lens.
